# EUROPEAN PATENT APPLICATION

(11) **EP 3 747 992 A1**
(43) Date of publication of application: **09.12.2020**
(21) Application number: 19178122.8
(22) Date of filing: 04.06.2019
(51) Int. Cl.: C12N 5/071, A61K 35/44

(54) **COMPOSITIONS AND PROCESS FOR INTEGRATING CELLS INTO EPITHELIUM**

(71) Applicant: Medizinische Hochschule Hannover, 30625 Hannover (DE)
(72) Inventor: MARTIN, Ulrich, 30625 Hannover (DE)
(74) Representative: Taruttis, Stefan Georg

(57) **Abstract**

The invention provides a combination of compositions comprising in a first composition 17β-estradiol as the active ingredient and, as a second composition, a suspension of cells for use in the treatment of functional defects of an epithelium, e.g. of an epithelium of a tissue, which tissue may be part of an organ.

## Description

The present invention relates to compositions for use in integrating cells into tissue, e.g. tissue of an organ, and to a process for integrating cells into extracorporeal epithelium of a tissue or organ, wherein the integrating cells differ in at least one characteristic from the cells of the epithelium, e.g. the integrating cells are of heterologous origin, are heterologous or autologous cells with a genetic or epigenetic alteration introduced, or are derived from a stem cell line, e.g. pluripotent stem cells (PSCs), e.g. induced PSC (iPSCs), generated from a biopsy of the patient, e.g. autologous iPSC, or heterologous stem cells, in each case optionally with a genetic alteration introduced. Preferably, the integrating cells are immunologically compatible to the epithelial tissue. The integrating cells can e.g. be epithelial cells including endothelial cells. From the integrating cells, cells which are derived from human embryos while destroying the embryo are excluded.

Herein, epithelium also includes endothelium, e.g. both epithelial layers and endothelial layers. The epithelium, e.g. endothelium, can be located on an internal or external surface of a tissue, which can be an organ.

Specifically, the invention relates to a combination of compositions including a suspension of cells for use in the treatment of epithelium, especially for use in the treatment of defective epithelium, especially for introducing integrating cells into the epithelium of a tissue, which may be an organ. The cells for use in the treatment of the epithelium are for integration into the epithelium. In addition to the use of the combination of compositions, which combination includes a suspension of cells, in the treatment of epithelial tissue, the invention also relates to a process for introducing integrating cells, e.g. epithelial and/or endothelial cells into epithelium, which may be endothelium, in order to produce an epithelium, which may be an endothelium, containing the integrating cells from the suspension in an integrated state. In the process, the epithelium may be part of an organ, which is preferably isolated from the body of a patient, e.g. the process preferably is an extracorporeal process. Generally, the integrating cells are used in suspension, e.g. in a single-cell or multicellular suspension. Prior to the use and prior to the process, the integrating cells are separate from the tissue containing the epithelium, e.g. separate from the patient to be treated. The suspension medium can be a cell cultivation medium, preferably a serum-free medium.

### State of the art

Groten et al., The FASEB Journal 19, No. 10, 1368 (2005) describe that 10 nM 17β-estradiol increases the permeability for FITC-labelled dextran of a cultivated monolayer of endothelial cells, namely human umbilical vein endothelial cells and uterine human microvascular endothelial cells.

WO 2016/203477 A1 describes a combination of compounds for use in conditioning a person for subsequent transplantation of progenitor cells, which combination includes a cell damaging agent and, for subsequent administration, an agent destroying resident stem cells.

EP 2 788 003 B1 describes a cell suspension from a mammalian fetal pulmonary tissue for use in treating or regenerating an epithelial, mesenchymal or endothelial tissue in a patient.

EP 3 034 087 B1 describes a cell suspension from a mammalian fetal pulmonary tissue for use in treating or regenerating an epithelial, mesenchymal or endothelial tissue in a patient who was pre-conditioned by a sublethal, lethal or supralethal conditioning protocol which includes a naphthalene treatment.

### Object of the invention

An object of the invention is to provide compositions for use in the treatment of epithelial tissue and a process suitable for integrating cells into epithelial tissue, e.g. for generating endothelial tissue containing the integrating cells within its endothelial tissue structure. Preferably, the compositions and the process do not have activity to destroy cells of the endothelial tissue to be treated. More preferably, the compositions and the process in addition to the cells in suspension use only natural compositions for disintegrating the existing epithelial cell layer as basis for integrating cells into an epithelial tissue.

### Description of the invention

The invention achieves the object by the features of the claims, especially by a combination of compositions comprising or consisting of a first composition containing or consisting of 17β-estradiol as the active ingredient in a buffer solution, and, as a second composition, a suspension of cells in a suspension medium, which combination of compositions is for use in the treatment of functional defects of an epithelium, e.g. of an epithelium of a tissue, which tissue may be part of or may be an organ, wherein the suspension of cells is for application at a time after the application of the 17β-estradiol to the epithelium, e.g. to the tissue containing the epithelium. The epithelium may e.g. be the epithelial layer or endothelial layer of the tissue, e.g. endothelium, e.g. of endothelialised vessels, or epithelia. The combination of compositions for use in the treatment of tissue containing epithelium, e.g. endothelium, comprises a first composition containing or consisting of 17β-estradiol as the active ingredient and a second composition comprising a suspension of cells, wherein the second composition is for contacting the epithelium of the tissue at a time after contacting the epithelium of the tissue with the first composition. Therein, the time between the contacting of the epithelium with the first composition and contacting the epithelium with the second composition can e.g. be at least 1 min, e.g. at least 5 min or at least 10 min, e.g. up to 3 h, up to 2 h, or up to 60 min or up to 45 min or up to 30 min. Alternatively, the epithelium can be contacted by the second composition immediately after removal of the first composition. Generally, the epithelium is contacted with the second composition separate from the first composition, preferably the first composition is removed from the epithelium prior to contacting the epithelium with the second composition. The first composition contains or consists of 17β-estradiol as the active ingredient at a concentration suitable for adjusting the concentration of 17β-estradiol in the medium contacting the epithelium to at least 1 µM, e.g. at least 10 µM, e.g. at least 20µM or at least 30 µM 17β-estradiol, e.g. at least 50 µM, or at least 75 µM or at least 100 µM or at least 150 µM, e.g. up to 300 µM, e.g. up to 250 µM, up to 200 µM, up to 150 µM or up to 130 µM, optionally 30 to 100 µM 17β-estradiol. Optionally, in the time after contacting the epithelium with the first composition, e.g. prior to contacting the epithelium with the second composition, the endothelium may be contacted with a third composition, which is free from 17β-estradiol and does not contain added cells.

The combination of compositions and the process, respectively, have the advantage of being adapted to maintain the cells of the original tissue containing the epithelium and to integrate the cells of the second composition, herein also termed integrating cells, as additional cells into the original endothelium. The additional cells contained in the resulting endothelium are provided by the second composition. The combination of compositions and the process, respectively, are suitable for use in generating a tissue having a closed epithelium, e.g. a closed epithelial cell layer comprising or consisting of the original epithelium with integrated cells of the suspension of cells. A further advantage of the combination of compositions and the process, respectively, is that it is adapted to generate a closed layer of the original epithelium having integrated cells originating from the suspension of cells within 2 to 10 h, e.g. 3 to 4 h, measured from contacting the tissue containing the epithelium with the first composition of the combination. The first composition comprising 17β-estradiol is preferably for contacting, preferably for perfusing or flushing, the tissue containing the epithelium for at least 5 min, e.g. up to 3 h, e.g. up to 90 min, up to 60 min or up to 45 min, e.g. 15 min to 3 h, e.g. 1 to 3 h, or 30 min to 2 h, preferably 20 to 30 min. The second composition is preferably for contacting, preferably for perfusing, the cavities, e.g. vessels or broncheoli, in the tissue lined by the epithelium. The second composition is e.g. contacted with the endothelium for at least 15 min, e.g. up to 3 h, e.g. 30 min to 2 h, or 45 min to 90 min, e.g. 20 to 30 min. The contacting of the epithelium can also be by contacting the epithelium with an aerosol of the first and/or the second composition, e.g. by inhalation into a lung as the epithelium of the tissue, respectively as the epithelium of the organ.

The cells of the suspension of cells can be cultivated cells or, less preferred, cells obtained from disintegration of a tissue. Herein, the cells of the suspension are also referred to as integrating cells, as the combination of compositions, and respectively the process, result in the integration of the cells of the second composition into the treated tissue containing the epithelium, which herein can include endothelium, and accordingly, epithelium herein is also referred to as epithelial and/or endothelial tissue, e.g. endothelialised vessels. Herein, integrating cells that are termed epithelial cells also include endothelial cells, e.g. epithelial cells can be epithelial cells only, or endothelial cells only, or a mixture of both epithelial cells and endothelial cells, of the second composition.

The combination of the compositions comprises or consists of a first composition comprising or consisting of 17β-estradiol as the active ingredient and, for application to the epithelium separate and at a time after application of the first composition, as a second composition a suspension of cells, and optionally a solution, e.g. a medium free from 17β-estradiol and free from cells, as a third composition, which is for use for contacting the epithelium between the step contacting the epithelium with the first composition and the step of contacting the epithelium with the second composition. Accordingly, a third composition which is a medium free from 17β-estradiol and free from cells is contained in the combination and is for contacting the tissue containing the epithelium subsequent to contacting the tissue containing the endothelium with the first composition and before contacting the tissue containing the endothelium with the second composition. The third composition can e.g. be used for contacting the epithelium for at least 1 min, e.g. at least 5 or at least 10 min. The third composition can e.g. be used for contacting the epithelium for up to 60 min, e.g. up to 30 min or up to 20 min.

The first composition contains 17β-estradiol at a concentration that is sufficient for adjusting the concentration of 17β-estradiol in the medium contacting the tissue containing the epithelium, wherein the medium can be a medium suitable to maintain the viability of the tissue containing the epithelium, e.g. a physiological buffer, a cell culture medium, an organ culture medium, or blood, and to support the effect of 17β-estradiol.

The second composition comprises cells in suspension, which preferably are epithelial and/or endothelial cells or progenitor cells of epithelial and/or of endothelial cells, e.g. a stem cell line, or pluripotent stem cells (PSC), e.g. induced PSC (iPSC), e.g. generated from a biopsy of the patient, e.g. homologous iPSC, or heterologous stem cells, in each case optionally with a genetic alteration introduced. The medium of the second composition, in which the cells are in suspension, can be a medium suitable to maintain the viability of the tissue containing the epithelium to be treated, and suitable to maintain the viability of the suspended cells, e.g. a cell culture medium, an organ culture medium, or blood, or a cell-free fraction of blood. Preferably, the combination of compositions is for use in the treatment of an epithelium contained in a tissue, wherein the epithelial tissue is perfused separately from the blood circulation of the patient from which the epithelial tissue originates. For example, the epithelial tissue for perfusion is separated from the blood circulation of a patient, and is connected to an artificial medium circulation system for artificial perfusion separate from the blood circulation of a patient.

Optionally, the epithelial tissue may be located within the patient or may be located external to the patient, wherein in each case the epithelial tissue preferably is perfused separately from the blood circulation of the patient.

Accordingly, the process preferably is a process in which the tissue containing the epithelium is perfused separately from the blood circulation of the patient from whom the tissue containing the epithelium originates, e.g. the process can be an extracorporeal process, e.g. in an ex vivo perfusion system system adapted to maintain the epithelium and the tissue containing it in a living state. Ex vivo perfusion systems are e.g. known for maintaining excised organs prior to transplantation or implantation into a patient.

The combination of compositions for use in the treatment of the epithelium is preferred for tissue containing the epithelium that is perfused by a medium separate from the blood circulation from a patient from which the epithelium, respectively the tissue containing the epithelium, originates. It has been found that the active component of the first composition, 17β-estradiol, when contacting the endothelium in the medium at the concentration of at least 1 µM, preferably at least 10 µM, or at least 20 µM, more preferably at least 30 µM, e.g. up to 300 µM, e.g. up to 250 µM or up to 200 µM, e.g. 30 to 150 µM, results in the formation of gaps within the epithelium which are sufficient for the integration of suspended cells into the epithelium. It has been found that the presence of 17β-estradiol at the concentration in the medium contacting, e.g. perfusing, the tissue containing the epithelium results in a loosening or disintegration of the original structure of the epithelium, and that cells contacted with this epithelium at a time subsequent to the contact with the first composition integrate into the epithelium of the tissue. It was found that merely contacting the epithelium at a time after contact with the first composition with a suspension of the cells is sufficient for their integration, e.g. by perfusing the tissue containing the epithelium with a suspension of cells.

Generally preferred, the cells in suspension are free from added 17β-estradiol, e.g. the medium of the suspension is free from 17β-estradiol.

It was found in *in vitro* tests that 3 to 5 h subsequent to contacting an epithelium with cells in suspension a closed endothelial layer was produced, containing both cells of the original epithelium and cells of the suspension of cells.

Preferably, the first composition is for contacting the epithelium, e.g. the tissue containing the epithelium, for a period of time of 5 min to 3 h, e.g. 15 min to 2 h, e.g. 10 min to 60 min, e.g. 20 to 40 min, e.g. 30 min, prior to contacting the epithelium, e.g. the tissue containing the epithelium with the cells in suspension. Optionally, the combination of compositions may comprise a third composition for application to the epithelium, e.g. to the tissue containing the epithelium, subsequent to the contact by first composition and prior to contacting the epithelium, respectively the tissue containing the epithelium, with the cells in suspension of the second composition, which third composition is free from 17β-estradiol, e.g. which third composition is a medium suitable for sustaining viability of the epithelium, e.g. a tissue culture medium, an organ culture medium, or a cell culture medium, preferably serum- free, or blood.

It was found that the combination of compositions is effective in resulting in the integration of cells of the second composition into the tissue containing the epithelium. Preferably the combination of compositions does not result in significant or sustained development of edema in the tissue containing the endothelium.

The combination of compositions for use in the treatment of tissue containing the epithelium as well as the process, respectively, according to the invention is suitable for producing a closed epithelial and/or endothelial cell layer which comprises both cells of the original epithelium and cells of the suspension of cells, wherein preferably in the closed epithelial and/or endothelial cell layer, the cells are connected by adherens junctions.

Generally, the epithelium is preferably characterized by containing adherens junctions. The epithelium, e.g. an epithelial or endothelial layer, may be part of a tissue, e.g. of an organ, which tissue can e.g. be lung tissue, e.g. a partial or complete lung, a blood vessel, an epithelial layer of the kidney or of the urinary tract.

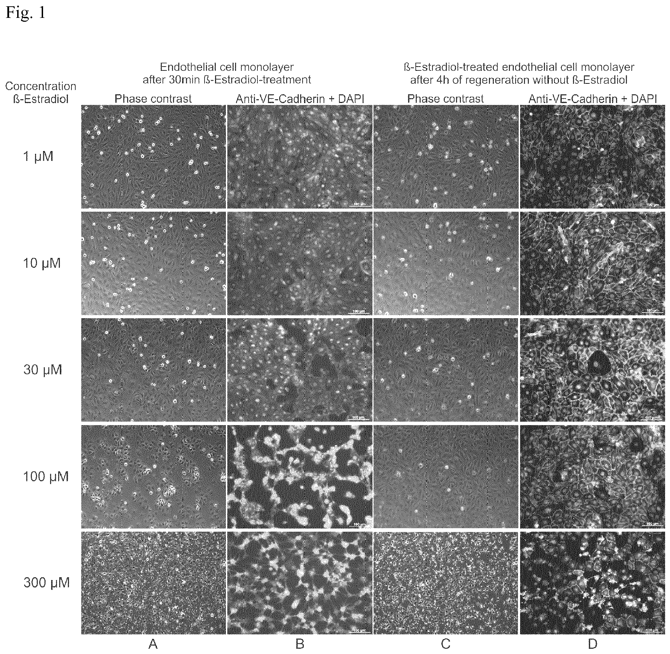

The invention is also described in greater detail with reference to the figures, which show in
- Fig. 1 microscopic pictures of monolayers of cultivated endothelial cells following contact with 17β-estradiol at different concentrations after 30 min, in column A in phase contrast and in column B after anti-VE-cadherin (green) and DAPI (blue) staining after 4 h regeneration in medium without 17β-estradiol subsequent to the incubation in the medium containing 17β-estradiol. C shows phase contrast, D shows anti-VE-cadherin and DAPI staining.

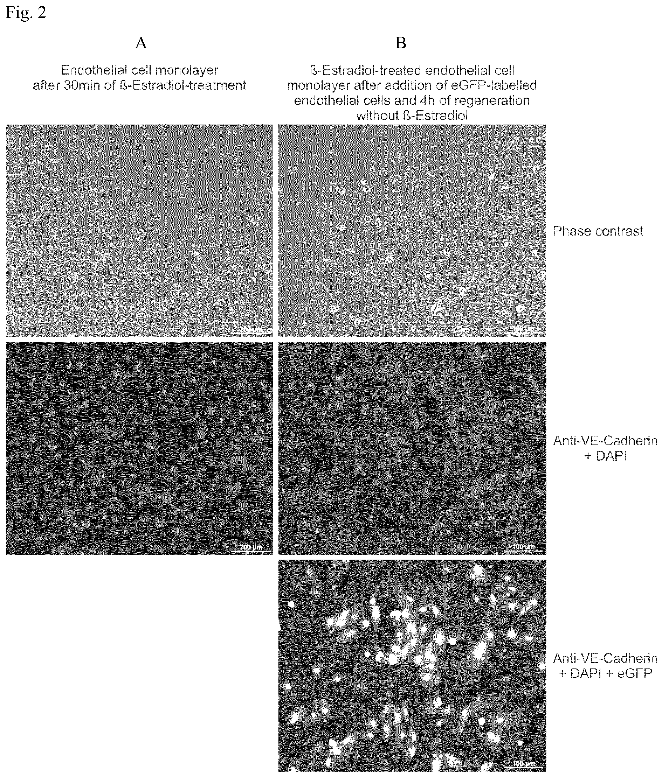

- Fig. 2 shows microscopic pictures of monolayers, in column A after 30 min in presence of 17β-estradiol, and in column B subsequent to additional contacting with cells in suspension, Upper row: phase contrast; middle row: anti-VE-cadherin staining (red) and DAPI staining (blue); lower row: anti-VE-cadherin staining (red), DAPI staining (blue) and detection newly integrated eGFP expressing endothelial cells (green).
In the figures, the scale bar is 100µm.

### Example: Integration of human primary venous endothelial cells into a venous endothelial tissue

As an example for an endothelium, human primary venous endothelial cells were cultivated in EGM-medium under cell culture conditions until reaching confluence. Then, the medium was removed from this exemplary epithelium and replaced by fresh EGM-medium containing 17β-estradiol in a concentration of 1 µM, 10 µM, 30 µM, 100 µM, or 300 µM. The concentrations of 17β-estradiol are also indicated in the rows of Fig. 1. Fig. 1 shows microscopic pictures of monolayers of the cultivated endothelial cells following the contact with 17β-estradiol at the concentrations after 30 min, in column A in phase contrast and in column B after immunological staining for anti-VE-cadherin and DAPI staining. This shows that the endothelial layer of venous endothelial cells was loosened, respectively shows disintegration after presence of at least 10 µM, preferably at least 30 µM 17β-estradiol, and at 100 µM and 300 µM 17β-estradiol for 30 min.

Subsequent to the presence of the 17β-estradiol in medium, this first composition was removed and replaced by EGM-medium without added 17β-estradiol, with subsequent incubation for 4 h under cell culture conditions. The result is depicted in Fig. 1 in column C in phase contrast, and in column D after immunological staining for VE-cadherin as an essential component of adherens junctions between cells, and DAPI staining for nuclei.

The results demonstrate that that after removal of 17β-estradiol a closed layer of the endothelial cells is re-established.

A confluent layer of human primary venous endothelial cells cultivated in EGM-medium was used as a representative for endothelium. This endothelial cell layer was contacted with a first composition containing 17β-estradiol in a concentration yielding 100 µM 17β-estradiol in fresh cell culture medium. After incubating this endothelial cell layer under cell culture conditions for 30 min in the presence of 100 µM 17β-estradiol, the medium was removed and replaced by a suspension of iPSC-derived endothelial cells that were genetically manipulated to constitutively express enhanced green fluorescent green protein (eGFP) in EGM-medium without added 17β-estradiol.

The results are depicted in Fig. 2, showing in column A that contacting the endothelial cell layer with 17β-estradiol within 30 min results in loosening of the cell layer and loss of VE-cadherin expression. The result of the subsequent 4h of incubation of the endothelial cell layer with eGFP-labelled endothelial cells in suspension is depicted in Fig. 2 in the right column B. This result shows that during reestablishment of VE-cadherin expression and adherens junctions, eGFP-labelled endothelial cells of the cell suspension efficiently integrate between the original endothelial cells of the epithelium, which here is an endothelium, and form a closed endothelial cell layer comprising both endothelial cells of the original endothelium formed by the endothelial cell layer and eGFP-labelled cells of the cell suspension.

## Claims

1. Combination of compositions for use in the treatment of epithelium, comprising a first composition containing 17β-estradiol as the active ingredient, and a second composition comprising a suspension of cells, wherein the second composition is for contacting the epithelium at a time after the contact with the first composition.

2. Combination of compositions for use in the treatment of epithelium according to claim 1, wherein the epithelium is an endothelium contained in a tissue or organ that is e.g. selected from lung, a blood vessel, kidney or urinary tract.

3. Combination of compositions for use in the treatment of epithelium according to one of the preceding claims, wherein the first composition and the second composition are for contacting the epithelium by perfusing, flushing, or contacting with an aerosol the tissue or organ containing the epithelium separate from the blood circulation of a patient from whom the tissue or organ containing the epithelium originates.

4. Combination of compositions for use in the treatment of epithelium according to one of the preceding claims, wherein the first composition contains 17β-estradiol in a concentration suitable for adjusting the concentration of 17β-estradiol in a medium contacting the epithelium to at least 1 µM 17β-estradiol.

5. Combination of compositions for use in the treatment of epithelium according to one of the preceding claims, wherein the first composition is for contacting the epithelium for 5 min to 3 h and the second composition is for subsequently contacting the epithelium for at least 15 min.

6. Combination of compositions for use in the treatment of epithelium according to one of the preceding claims, wherein the cells of the second composition are epithelial and/or endothelial cells or progenitor cells of epithelial and/or of endothelial cells, stem cells or progenitor cells, pluripotent stem cells (PSC), induced PSC (iPSC), homologous cells generated from a biopsy of the patient, and/or heterologous stem cells, in each case optionally with a genetic alteration introduced.

7. Combination of compositions for use in the treatment of epithelium according to one of the preceding claims, comprising a third composition which is a medium free from 17β-estradiol and free from cells, for contacting the tissue containing the epithelium subsequent to contacting the tissue containing the epithelium with the first composition and before contacting the tissue containing the epithelium with the second composition.

8. Combination of compositions for use in the treatment of epithelium according to one of the preceding claims, wherein the time after the contact with the first composition is at least 1 min.

9. Combination of compositions for use in the treatment of epithelium according to one of the preceding claims, wherein the epithelium is an endothelium.

10. Combination of compositions for use in the treatment of epithelium according to one of the preceding claims, wherein the epithelium is the epithelial and/or endothelial layer of a blood vessel, of a lung, of a kidney, or of the urinary tract.

11. Process for treatment of an extracorporeal epithelium, comprising contacting the epithelium with a first composition containing 17β-estradiol as the active ingredient, and after contacting the epithelium with the first composition, contacting the epithelium with a second composition comprising a suspension of cells.

12. Process according to claim 11, wherein the extracorporeal endothelium is comprised in an extracorporeal tissue or organ, which is located outside of the body of a patient and artificially perfusing the extracorporeal tissue or organ separate from the blood circulation of the patient.

13. Process according to one of claims 11 to 12, wherein the epithelium is contacted with the first composition for at least 5 min and is subsequently contacted with the second composition for at least 15 min.

14. Process according to one of claims 11 to 13, wherein subsequent to being contacted with the first composition and prior to being contacted with the second composition, the epithelium is contacted with a third composition that is free from 17β-estradiol and free from added cells.

15. Process according to one of claims 11 to 14, wherein the extracorporeal tissue or organ is isolated from a lung or is a lung, a portion of the respiratory tract including trachea, mouth and nose, a kidney, a part of the urinary tract, a part of the small intestine, colon, stomach, esophagus, bile duct, or pancreas.
